# EUROPEAN PATENT APPLICATION

(11) **EP 4 230 266 A1**
(43) Date of publication of application: **23.08.2023**
(21) Application number: 22157087.2
(22) Date of filing: 16.02.2022
(51) Int. Cl.: A61Q 19/10, A61K 8/25, A61K 8/64, A61K 8/73, A61K 8/81, A61K 8/26, A61K 8/90

(54) **COMPOSITION AND PROCESS FOR DECONTAMINATING POLYCYCLIC AROMATIC HYDROCARBONS AND SOOT**

(71) Applicant: DermaPurge GmbH, 01069 Dresden (DE)
(72) Inventor: Schnepf, Max Julian, 01099 Dresden (DE); Schubert, Jonas, 01099 Dresden (DE)
(74) Representative: Maikowski & Ninnemann Patentanwälte Partnerschaft mbB

(57) **Abstract**

Composition for decontaminating polycyclic aromatic hydrocarbons and soot from human skin, comprising
(A) water;
(B) at least one phyllosilicate;
(C) at least one water-soluble or water-dispersible polymer; and
(D) optionally at least one thickening agent;
wherein the composition does not contain charcoal and/or graphite and/or another carbon-based powder.

## Description

### FIELD OF THE INVENTION

The invention relates to a composition and a process for decontaminating polycyclic aromatic hydrocarbons and soot from any surface, preferably from human skin.

### BACKGROUND

Polycyclic aromatic hydrocarbons (PAHs) are formed during the combustion of wood-, coal- or petroleum-based products. They include carcinogenic substances, the human contact with which should be strictly avoided. In some fields, however, humans such as chimney sweepers may come into direct contact with PAHs when cleaning and maintaining chimneys. A possible resulting disease is "chimney sweep cancer" such as scrotal carcinoma - a skin cancer of the testicle. Firefighters are also exposed to PAHs when they fight fires. In the petrochemical industry, these hazardous substances may occur as a by-product. Particularly during plant maintenance, they may pose a problem for occupational safety and the health of cleaning personnel who come into direct contact with the PAHs.

Typically, said hazardous PAHs are included in soot.

Currently, in daily practice to remove the hazardous substances from their skin, workers resort to water, soap and surfactant-containing washing pastes.

However, cleansing compositions often include penetration enhancers, i.e., substances that promote the penetration of compounds through the human skin. Thus, the workers clean their skin superficially, but the hazardous substances may still disadvantageously pass through the skin. Moreover, even this superficial cleaning may be of low effectiveness.

The working mechanism of penetration enhancers is, e.g., explained in Thong H.-Y., Zhai H., Maibach, H.I., Percutaneous Penetration Enhancers: An Overview. Skin Pharmacol Physiol. 2007; 20: 272-282; Dragicevic, N., Maibach, H.I., Percutaneous Penetration Enhancers, Chemical Methods in Penetration Enhancement, Springer-Verlag Berlin Heidelberg 2016.

WO 2021/214209 provides a composition for decontaminating hazardous agents from skin, such as nanoparticles, microplastic particles and viruses. The composition comprises from 1 to 40 % by weight of at least one water-soluble polymer, from 1 to 40 % by weight of at least one phyllosilicate, from 1 to 30 % by weight charcoal and/or graphite and/or another carbon-based powder, and water; and wherein said composition for decontamination of skin does not comprise penetration enhancers.

### OBJECT OF THE INVENTION

The object of the present invention is to provide a composition suitable for decontamination, i.e., suitable for removing a PAH or soot, preferably PAH and soot, from any surface, preferably from a human skin.

In a preferred embodiment, the composition should be void of penetration enhancers.

### SUMMARY

This object could be achieved with a composition as defined in the appended claims.

Specifically, the invention relates to the following items:
1. Composition, comprising
   (A) water;
   (B) at least one phyllosilicate;
   (C) at least one water-soluble or water-dispersible polymer; and
   (D) optionally at least one thickening agent;
   wherein the composition contains less than 1 % by weight charcoal and/or graphite and/or another carbon-based powder, preferably wherein the composition does not contain charcoal and/or graphite and/or another carbon-based powder.
2. Composition of item 1, wherein
   (C) is or comprises at least one water-soluble or water-dispersible polyether or protein or a combination thereof.
3. Composition of item 1 or 2, wherein
   (D) the thickening agent is selected from the group consisting of polysaccharide, cellulose ether, and polymer based on polyacrylic acid.
4. Composition of item 1, wherein
   (B) is or comprises, preferably is, a combination of at least two different phyllosilicates; preferably
   wherein
   (B) is or comprises, preferably is, a sodium aluminium silicate or an aluminium silicate or a combination thereof.
5. Composition of any one of the preceding items, wherein
   (B) is or comprises, preferably is, bentonite or kaolinite or a combination thereof.
6. Composition of any one of the preceding items, wherein
   (C) is or comprises, preferably is, a poloxamer or a poloxamine or a soybean protein or a combination of two or more thereof.
7. Composition of item 6, wherein the poloxamer has the CAS no. 691397-13-4 or 9003-11-6 or the soybean protein is soybean protein isolate.
8. Composition of any one of the preceding items, wherein
   (D) is or comprises, preferably is, a cellulose ether or a polysaccharide or a combination thereof.
9. Composition of any one of the preceding items, wherein
   (D) is or comprises, preferably is, a polysaccharide selected from xanthan, chitosan, dextran, guar gum, polygeline, pectin and carrageenan.
10. Composition of any one of the preceding items, comprising
   (A) from 20 to 90 % by weight;
   (B) from 1 to 40 % by weight;
   (C) from 1 to 40 % by weight;
   (D) from 0 to 10 % by weight;
   based on the total weight of (A) to (D) (100 % by weight); preferably comprising
   (A) from 30 to 80 % by weight;
   (B) from 5 to 30 % by weight;
   (C) from 5 to 30 % by weight;
   (D) from 0 to 10 % by weight;
   based on the total weight of (A) to (D) (100 % by weight).
11. Cleaning composition comprising or consisting of the composition as defined in any one of the preceding items.
12. Cleaning composition of item 11, comprising from 40 to 100 % by weight of the composition defined in any one of items 1 to 10.
13. Use of a composition as defined in any one of the preceding items for removing polycyclic aromatic hydrocarbons or soot or polycyclic aromatic hydrocarbons and soot from human skin.
14. Method of cleaning human skin, wherein said skin is contaminated with a polycyclic aromatic hydrocarbon or soot or polycyclic aromatic hydrocarbons and soot, comprising steps (A) and (B) and optionally step (C):
   (A) contacting said skin with a composition as defined in any one of items 1 to 12;
   (B) rinsing the contacted skin of step (A) with water;
   (C) optionally repeating steps (A) and (B).
15. Use of item 13 or method of item 14, wherein said polycyclic aromatic hydrocarbon is comprised in soot.

### BRIEF DESCRIPTION OF THE FIGURE

The figure shows:

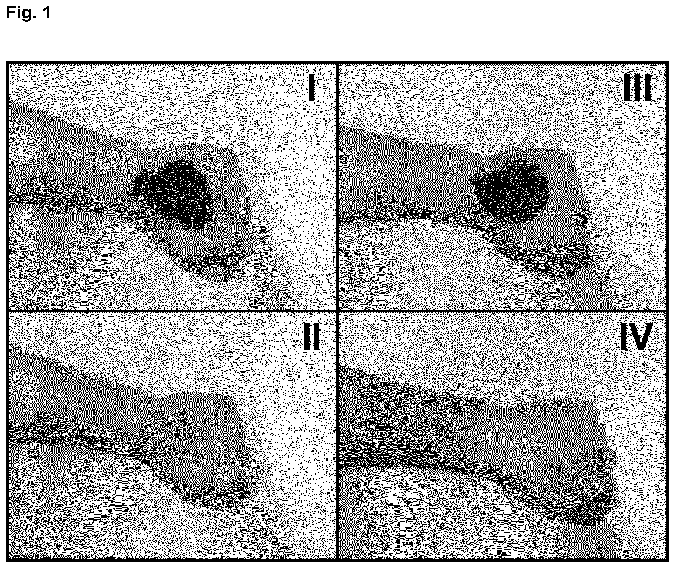

**Fig. 1** a graphic representation of the differences in removing soot from the skin, either by a composition as disclosed in WO 2021/214209 (I+II) and by a composition according to the invention (III+IV).

### DETAILED DESCRIPTION

### First aspect: Composition according to the invention

In a first aspect, the invention relates to a composition comprising
(A) water;
(B) at least one phyllosilicate;
(C) at least one water-soluble or water-dispersible polymer; and
(D) optionally at least one thickening agent;
wherein the composition contains less than 1 % by weight charcoal and/or graphite and/or another carbon-based powder, preferably wherein the composition does not contain charcoal and/or graphite and/or another carbon-based powder.

In a preferred embodiment, (C) is or comprises at least one water-soluble or water-dispersible polyether or protein or a combination thereof.

In a further preferred embodiment, the thickening agent (D) is selected from the group consisting of polysaccharide, cellulose ether, and polymer based on polyacrylic acid.

Therefore the invention relates to a composition comprising
(A) water;
(B) at least one phyllosilicate;
(C) at least one water-soluble or water-dispersible polyether or protein or a combination thereof;
(D) optionally at least one agent selected from the group consisting of polysaccharide, cellulose ether, and polymer based on polyacrylic acid;
wherein the composition contains less than 1 % by weight charcoal and/or graphite and/or another carbon-based powder, preferably wherein the composition does not contain charcoal and/or graphite and/or another carbon-based powder.

Preferably the composition does not contain charcoal and/or graphite and/or another carbon-based powder.

Preferably the composition does not contain a substance that may act as a penetration enhancer.

Preferably the composition contains less than 1 % by weight charcoal and/or graphite and/or another carbon-based powder, preferably wherein the composition does not contain charcoal and/or graphite and/or another carbon-based powder, and the composition does not contain a substance that may act as a penetration enhancer.

Further, the invention relates to a composition consisting of
(A) water;
(B) at least one phyllosilicate;
(C) at least one water-soluble or water-dispersible polymer; and
(D) optionally at least one thickening agent;
wherein the composition contains less than 1 % by weight charcoal and/or graphite and/or another carbon-based powder, preferably wherein the composition does not contain charcoal and/or graphite and/or another carbon-based powder.

Further, the invention relates to a composition consisting of
(A) water;
(B) at least one phyllosilicate;
(C) at least one water-soluble or water-dispersible polyether or protein or a combination thereof;
(D) optionally at least one agent selected from the group consisting of polysaccharide, cellulose ether, and polymer based on polyacrylic acid;
wherein the composition contains less than 1 % by weight charcoal and/or graphite and/or another carbon-based powder, preferably wherein the composition does not contain charcoal and/or graphite and/or another carbon-based powder.

### (A) Water

The term "water" as used herein in (A) encompasses tab water, process water, demineralized water, distilled water, and drinking water.

### (B) Phyllosilicate

The term "phyllosilicate" as used herein in (B) encompasses sheet silicate minerals. Such sheet silicates are typically formed by parallel sheets of silicate tetrahedra with Si₂O₅ or a 2:5 ratio.

Examples of phyllosilicates suitable for use in the composition of the invention are ajoite, allophane, annite, apophyllite, apophyllite-(kf), bentonite, biotite, bowenite, brammallite, carletonite, caryopilite, cavansite, chamosite, chapmanite, chrysocolla, clay, clay mineral, clintonite, cymrite, delessite, dickite, ekanite, ephesite, expanded clay aggregate, fraipontite, franklinphilite, fuchsite, greenalite, gyrolite, halloysite, hisingerite, imogolite, kampfite, kaolinite, kegelite, kerolite, lepidolite, macaulayite, macdonaldite, magadiite, medicinal clay, meerschaum pipe, metal clay, mica, minnesotaite, nelenite, neptunite, okenite, organoclay, pentagonite, petalite, phlogopite, pimelite, pyrophyllite, sanbornite, searlesite, sepiolite, seraphinite, sericite, sericitic alteration, siderophyllite, soapstone, stilpnomelane, talc, thuringite, tumchaite, tuperssuatsiaite, ussingite, zakharovite, and zussmanite.

Preferably, the at least one phyllosilicate comprised in the composition of the invention is selected from the group consisting of kaolin, bentonite, vermiculite, talc, mica, sepiolite, palygorskite, saponite, hectorite, smectite, montmorillonite K-10, aquamont-CA, illite, montmorillonite KSF and aquamont B, or a mixture of two or more thereof.

Preferably, (B) is a combination of at least two different phyllosilicates.

Further preferably, (B) is selected from a sodium aluminium silicate and an aluminium silicate or a combination thereof.

Further preferably, (B) is selected from bentonite and kaolinite or a combination thereof.

The term "kaolin" as used herein is synonymously used with the terms "kaolinite" or "China clay".

### (C) Water-soluble or water-dispersible polymer

The term "at least one water-soluble or water-dispersible polymer" as used herein in (C) encompasses any water-soluble or water-dispersible polymer.

The term "water-dispersible polymer" as used herein in (C) encompasses the term "water-emulsifiable".

Preferably, the polymer is water-soluble.

The term "water-soluble" as used herein encompasses a solubility of at least 30 wt.-% in water at 25°C, i.e., at least 30 parts of polymer and 70 parts of water form a solution.

The at least one water-soluble or water-dispersible polymer as used herein in (C) is or comprises at least one water-soluble or water-dispersible polyether or protein or a combination thereof. Specific examples for the at least one water-soluble or water-dispersible polymer include poly(N-isopropylacrylamide), polyacrylic acid, chitosan, dextran, guar gum, polyethylene imine, polyacryl amide, polyphosphate (sodium polyphosphate), polyphosphazene, or mixtures thereof

The term "at least one water-soluble or water-dispersible polyether or protein or a combination thereof" as used herein in (C) encompasses any water-soluble or water-dispersible polyether and/or protein.

In a preferred embodiment, the polyether or protein is water-soluble.

The term "water-soluble" as used herein encompasses a solubility of at least 30 wt.-% in water at 25 °C, i.e., at least 30 parts of polyether or protein and 70 parts of water form a solution.

The term "polyether" as used herein encompasses structures such as -R¹-OR²-O-R³-O-, wherein R¹, R², R³ denote independently from one another any organic residues. Such polyethers are well-known in the art.

Preferred polyethers are derived from poly(ethylene glycol) (PEO) and/or poly(propylene glycol) (PPO).

Specifically preferred polyethers are selected from the group consisting of a poly(ethylene glycol), a poly(propylene glycol), a poloxamer, and a poloxamine.

In a particularly preferred embodiment, the water-soluble or water-dispersible polymer is a poloxamer or a poloxamine.

Poloxamers are non-ionic triblock copolymers composed of a central hydrophobic chain of polyoxypropylene (poly(propylene oxide)) flanked by two hydrophilic chains of polyoxyethylene (poly(ethylene oxide)). Poloxamers are also known by the trade names Pluronic^{®}, Kolliphor^{®}, Synperonic^{®} and Lutrol^{®}.

Thus, poloxamers are ABA-type co-polymers of poly(ethylene oxide) (PEO = A) and poly(propylene oxide) (PPO = B). For example, Kolliphor^{®} P 407 indicates a poloxamer with approx. 70% m/m PEO and approximately an average molecular weight of PPO of 4000.

Depending on the type of poloxamers, it can act as penetration enhancer or reduce the penetration. Poloxamer 188, Poloxamer 401 are known to enhance the penetration of substances, whereas Poloxamer 407 (CAS no. 691397-13-4), Poloxamer 184 (CAS no. 9003-11-6), Poloxamer 304, Poloxamer 338 (CAS no. 9003-11-6) or Poloxamer 904 reduce the penetration of substances through the skin (Bruce J. Aungst, Nancy J. Rogers and Eli Shefter, Enhancement of naloxone penetration through human skin in vitro using fatty acids, fatty alcohols, surfactants, sulfoxides and amides, International Journal of Pharmaceutics, 33 (1986) 225-234; Markus J. Cappel and Jörg Kreuter, Effect of nonionic surfactants on transdermal drug delivery: II. Poloxamer and poloxamer surfactants, International Journal of Pharmaceutics, 69 (1991) 155-167).

The use of a poloxamer which reduces the penetration of substances through skin is preferred.

Thus, the use of one or more poloxamers having the CAS nos. 691397-13-4 or 9003-11-6 is preferred.

Likewise, the use of poloxamines is preferred.

Poloxamines are block copolymers of ethylene oxide and propylene oxide which additionally contain ethylenediamine.

The protein may be an animal protein or a plant protein.

In one embodiment, the protein is bovine serum albumin (BSA) or a soybean protein.

Soybean proteins are e.g., known under the term "soybean protein isolate".

### (D) Thickening agent

The compounds optionally used in (D) of the composition according to the invention are thickening agents, i.e., any agent that is suitable to increase the viscosity when added to the composition comprising (A), (B), and (C) or consisting of (A), (B), and (C).

The term "agent" as used herein encompasses the term "compound".

The term "thickening agent" as used herein in (D) preferably encompasses any agent that has shear-thinning properties in water, i.e., it reduces viscosity when being subjected to shear stress.

According to the invention, the composition may optionally comprise a thickening agent selected from polysaccharide, cellulose ether, polymer based on polyacrylic acid, or any combination of two or more thereof.

The polysaccharide is selected from xanthan, chitosan, dextran, guar gum, polygeline, pectin and carrageenan.

The cellulose ether is selected from methyl cellulose, carboxymethyl cellulose, hypromellose, ethyl cellulose, ethyl methyl cellulose, hydroxyethyl cellulose, hydroxypropyl cellulose, hydroxyethyl methyl cellulose, and ethyl hydroxyethyl cellulose, or a mixture thereof.

The polymer based on polyacrylic acid is a carbomer, a poly(N-isopropylacrylamide), or a polyacryl amide.

The term "carbomer" as used herein encompasses homopolymeric polyacrylic acids.

The term "acrylic" as used herein encompasses the term "methacrylic".

(D) may also comprise further polymers such as polyoxazoline and polyethylene imine, polymers such as polyphosphate (sodium polyphosphate) and polyphosphazenes.

Preferably, (D) is selected from a cellulose ether and a polysaccharide.

In a particularly, (D) is selected from carboxymethyl cellulose and xanthan.

The composition contains less than 1 % by weight charcoal and/or graphite and/or another carbon-based powder

According to the invention, the composition contains less than 1 % by weight charcoal and/or graphite and/or another carbon-based powder.

Preferably, the composition does not contain charcoal and/or graphite and/or another carbon-based powder

WO 2021/214209 discloses that exemplary charcoal is activated carbon, wherein said activated carbon is preferably made from hard coal, bituminated coal, coconut or peat and/or wherein said other carbon based powder is made from fruit shell or stone powder such as Olea Europaea Seed Powder, Prunus Amygdalus Dulcis (Sweet Almond) Shell Powder, Prunus Persica (Peach) Seed Powder, Pistacia Vera (Pistachio) Shell Powder, Persea Gratissima (Avocado) Extract, Prunus Armeniaca Seed Powder, Argania Spinosa Kernel Extract, Juglans Regia Shell Powder.

### Preferred Compositions

The composition of the invention may be provided as relatively diluted aqueous solution as well as a concentrated solution, respectively as a relatively diluted aqueous formulation as well as a concentrated formulation.

In one embodiment, the amount of (A) water in the composition is from 20 to 90 % by weight, based on the total amount of (A) to (D) (100 % by weight)

Beneficial results in the removal of hazardous compounds such as PAH or soot from skin have been achieved, when the composition of the invention comprises from 2 to 40 % by weight of at least one phyllosilicate, in particular bentonite. Preferably, the amount of the at least one phyllosilicate or a mixture of phyllosilicates, in particular bentonite or mixtures including bentonite, in the composition of the invention is 5 wt. % or higher, e.g. in the range between 5 % by weight and 40 % by weight or 8 % by weight and 30 % by weight or 10 % by weight and 30 % by weight or 16 % by weight and 30 % by weight, preferably between 16 % by weight and 25 % by weight or 16 % by weight and 20 % by weight. Such compositions typically allow the removal of more than 85 %, and up to 100 % of the PAH and soot from contaminated skin.

Preferably, the amount of compound (D) in the composition of the invention is in the range of 0 up to 10 % by weight, more preferably up to 0.1 %, 0.2 %, 0.3 %, 0.4 %, 0.5%, 0.6 %, 0.7 %, 0.8%. 0.9 %, 1 %, 1.1 %, 1.2 %, 1.3 %, 1.4 %, 1.5 %, 1.6 %, 1.7 %, 1.8 %, 1.9 %, 2.0 %,2.1 %,2.2 %, 2.3 %, 2.4 %,2.5 %, 2.6 %,2.7 %, 2.8 %, 2.9 %, 3.0 %, 3.5 %, 4.0 %, 4.5 %, 5 %, 5.5 %, 6 %, 6.5 %, 7 %, 7.5 %, 8 %, 8.5 %, 9 %, 9.5 % or 10 % by weight.

In one embodiment, the invention relates to a composition, comprising
(A) from 20 to 90 % by weight;
(B) from 1 to 40 % by weight;
(C) from 1 to 40 % by weight;
(D) from 0 to 10 % by weight;
based on the total weight of (A) to (D) (100 % by weight).

In another embodiment, the composition comprises
(A) from 30 to 80 % by weight;
(B) from 5 to 30 % by weight;
(C) from 5 to 30 % by weight;
(D) from 0 to 10 % by weight;
based on the total weight of (A) to (D) (100 % by weight).

### (E) Preservatives

In one embodiment, the composition according to the invention is sterile and preservative-free.

In other embodiments, compositions of the present invention optionally comprise a preservative.

In a preferred embodiment, a preservative is a paraben-free preservative.

The term "parabens" as used herein encompasses a series of parahydroxybenzoates or esters of parahydroxybenzoic acid. Examples of parabens include methylparaben, ethylparaben, propylparaben, butylparaben, heptylparaben, isobutylparaben, isopropylparaben, benzylparaben, and their sodium salts.

Exemplary paraben-free preservatives suitable for use in the present invention include phenethyl alcohol, phenoxyethanol, a sorbate, potassium sorbate, sodium sorbate, sorbic acid, sodium benzoate, benzoic acid, oleuropein, cranberry extract, *Lactobacillus* ferment, *Usnea barbata* extract, pomegranate extract, *Populus tremuloides* bark extract, resveratrol, anisic acid, sodium anisate, phenyl propanol, undecylenic acid, sorbitan caprylate, sodium polyphosphate, sodium phosphate, disodium phosphate, trisodium phosphate, disodium pyrophosphate, tetrasodium pyrophospahteor any combination thereof.

The amount of the preservatives in the composition of the invention is preferably up to 10 % by weight, more preferably up to 0.1 %, 0.2 %, 0.3 %, 0.4 %, 0.5 %, 0.6 %, 0.7 %, 0.8 %, 0.9 %, 1.0 %,2.0 %, 3.0 %,4.0 %, 5.0 %,6.0 %,7.0 %, 8.0 %, 9.0 % or 10 % per weight, based on the total amount of (A) to (E) (= 100 % by weight).

### (F) Further additives

In a further embodiment, the composition according to the invention for decontaminating skin may further comprise anti-inflammatory agents and agents beneficial for the skin barrier function, such as one or more agents selected from schizophyllan, glucomannan, polycitronellol and polycitronellol acetate. Said anti-inflammatory agents and agents beneficial for the skin barrier function are comprised

in the composition of the invention preferably in range from 0.05 to 2 % by weight, such as 0.05 %, 0.25 %, 0.5 %, 0.75 %, 1.0 %, 1.25 %, 1.5 %, 1.75 % or 2.0 % by weight.

In a further embodiment, the composition for decontaminating skin according to the invention may further comprise buffering agents, such as acids or bases to adjust the pH, such as one or more agents selected from hydrochloric acid, phosphoric acid, sodium hydroxide, sodium carbonate, sodium hydrogen carbonate, sulfuric acid and nitric acid. Said buffering agents are comprised in the composition of the invention preferably in range from 0 to 2.5 % by weight Q.S (quantum satis), such as 0.05 %, 0.25 %, 0.5 %, 0.75 %, 1.0 %, 1.25 %, 1.5 %, 1.75 %, 2.0 %, 2.25 % or 2.5 % Q.S.

In a further preferred embodiment, the pH of the composition is balanced in the range from 4.1 to 5.6, such 4.1, 4.2, 4.3, 4.4, 4.5, 4.6, 4.7, 4.8, 4.9, 5.0, 5.1, 5.2, 5.3, 5.4, 5.5 or 5.6.

Water is primarily used as a solvent in the composition of the invention. Preferably, the ingredients described herein are dissolved. The water also forms emulsions in which lipophilic and hydrophilic components of the composition are combined to form creams and lotions. Preferably the water has been sterilized before its use to manufacture the composition of the invention. The final amount of water comprised in the composition of the invention is calculated based on the sum of the amounts of the other ingredients. The sum amounts of the other ingredients are calculated in % by weight and the difference to 100 % per weight of the composition of the invention is compensated with water.

A typical amount of water comprised in the composition of the invention is in the range between 20 and 90 % by weight, preferably between 30 and 80 % by weight, and more preferably between 50 to 70 % by weight.

In a further preferred embodiment, the composition of the invention is formulated such that it does not contain substances that are penetration enhancers such as, e.g., surfactants. Therefore, in such preferred formulation, the composition of the invention is particularly advantageous, because the composition itself does not penetrate into and/or through the skin. A further advantage of such composition is that it does not affect the barrier function of the skin. In particular, such composition does not widen the pores of the skin, as this favors the diffusion of hazardous substances through the skin. Widening of skin pores has for example been recognized with conventional soap. Accordingly, the composition of the invention when formulated preferably with a poloxamer having the CAS nos. 691397-13-4 or 9003-11-6 does not facilitate the penetration of PAH or soot into and through the skin. Also, after treating a skin area which is contaminated with PAH, with the composition of the invention, there is no need for a subsequent skin care, because the skin is not negatively affected.

The subject-matter defined herein for the first aspect of the invention apply for all further aspects of the invention as well.

### Second aspect: Cleansing composition

According to a second aspect, the invention relates to a cleaning composition (cleansing composition) comprising or consisting of the composition as defined in the first aspect including any embodiment defined therein.

In one embodiment, the cleaning composition comprises from 40 to 100 % by weight of the composition defined in the first aspect including any embodiment defined therein.

The cleaning composition of the invention preferably is provided as a formulation that allows an easy administration of the composition on contaminated skin areas. Typical formulations, which fulfill this purpose, are creams, gels, pastes or lotions.

The term "lotion" as used herein encompasses a low-viscosity topical preparation intended for application to the skin. Lotions may be applied to human skin with bare hands, a brush, a clean cloth, or cotton wool.

By contrast, creams and gels have higher viscosity, typically due to lower water content or a higher amount of a thickening agent.

The term "cream" as used herein encompasses semi-solid emulsions of oil and water.

The term "gel" as used herein encompasses a substantially cross-linked system in the form of a semi-solid or even solid form, and which exhibits no flow when being in the steady state.

The subject-matter defined herein for the second aspect of the invention apply for all further aspects of the invention as well.

Third aspect: Use of a composition for removing polycyclic aromatic hydrocarbons from

### human skin

According to the third aspect, the invention relates to the use of a composition as defined in the first aspect or the second aspect or any embodiment defined therein for removing polycyclic aromatic hydrocarbons from a surface.

In a particularly preferred embodiment, the surface is human skin.

In a preferred embodiment, said polycyclic aromatic hydrocarbon is comprised in soot.

The term "soot" as used herein encompasses terms such as "carbon black" or "unburned carbon".

When used, the composition thus does not only remove the PAH being contained in the soot, but also the soot as such. This is a particular advantage.

The subject-matter defined herein for the third aspect of the invention apply for all further aspects of the invention as well.

### Fourth aspect: Method of cleaning human skin, wherein said skin is contaminated with a polycyclic aromatic hydrocarbon

According to a fourth aspect, the invention relates to a method of cleaning human skin, wherein said skin is contaminated with a polycyclic aromatic hydrocarbon, comprising steps (A) and (B) and optionally step (C):
(A) contacting said skin with a composition as defined in the first or second aspect;
(B) rinsing the contacted skin of step (A) with water;
(C) optionally repeating steps (A) and (B).

In a preferred embodiment, said polycyclic aromatic hydrocarbon is comprised in soot.

The subject-matter defined herein for the fourth aspect of the invention apply for all further aspects of the invention as well.

### EXAMPLES

### Example 1: General manufacturing process of the decontamination composition

In a first step, at least one water-soluble polymer according to (B) and optionally an agent according to (D) were mixed with purified water (A). Then, the at least one phyllosilicate according to (B) was homogenized and after homogenization added to the mixture of the at least one water-soluble polymer (B) and optionally the agent (D) in water. The mixture was thoroughly stirred until homogenization.

Further described is the representative manufacture of 100 g of a gel:

5 g of Soybean protein isolate were mixed with 75 g of purified water (18.2 MΩ). 10 g of bentonite and 10 g of kaolin were homogenized and then added to the mixture of the protein isolate in water. The completed mixture was thoroughly stirred until homogenization.

### Example 2: General design of the decontamination experiments

Decontamination experiments were performed with porcine skin.

### Preparation of porcine skin

Porcine skin was prepared as follows:

Pigs of the German Landrace are used, aged between 5 and 8 months. The age, sex and weight of each donor animal were documented in the test protocol.

Pieces of skin of approximately 10 cm x 10 cm were removed from the area of the lateral abdominal wall with the help of a scalpel. A distance of at least 5 cm from the spinal column, ribs and the middle of the abdomen were maintained. The pieces of skin were wrapped in surgical drapes to avoid contamination with subcutaneous fatty tissue. The transport to the laboratory was carried out in freezer bags in a cool box at approx. 4-8 °C on cooling batteries or ice.

First, the skin surface was cleaned with lukewarm water. Then the preparation base was covered with a layer of aluminium foil and above that a layer of absorbent material (e.g., Zemuko universal compress). A piece of skin was placed on top of this with the stratum corneum side down. Artery clamps were attached to the four corners of the skin, and the skin was stretched by means of the rubber bands. Alternatively, the skin was fixed with four needles which were inserted through the skin corners into a polystyrene pad. The subcutaneous fatty tissue was grasped with surgical tweezers and separated directly below the dermis with a scalpel. Care had to be taken that no contact of the surface with subcutaneous fat occurred. Due to the risk of contamination, the outermost 5 mm in the marginal area had to be discarded after the subcutaneous fat tissue had been dissected. The prepared skin was then placed on the aluminum foil with anatomical tweezers, smoothed if necessary, and wrapped in aluminum foil and placed in a freezer bag. The freezer bag was sealed as airtight as possible. The skin was frozen at -20 °C to -30 °C in the freezer on a flat surface. For further use, the samples were stored in the freezer for at least 24 hours. The maximum storage period was 6 months. A documentation of the storage period had to be provided.

Before use in the experiments, the skin pieces were hydrated. For hydration, a beaker with acceptor medium was tempered to 32 °C. The skin pieces were then placed in the beaker and completely covered by the acceptor medium.

### Decontamination experiments

Skin samples were contaminated with either fluorene (f) or benzo[a]pyrene (b), which are typical model systems for PAH.

Stock solutions were prepared as follows:
(f) = fluorene: 2.2 mg/mL in chloroform
(b) = benzo[a]pyrene: 3.3mg/mL in chloroform

Due to their fluorescence, the PAHs were quantitatively well detectable. After an exposure time of 30 seconds after contamination, appropriate countermeasures were taken. These were thorough rubbing with the composition of the invention and subsequent washing with cold water. All pieces of skin were examined in a fluorescence spectrometer.

The removal of soot was tested optically and documented via photographs. Results are shown in Tables 1-2 and Figure 1.

### Example 3: Screening experiments

Compositions were prepared as described in Example 1. Efficacy tests were performed in triplicate. The residual amount to 100 % by weight in the table represents water.

Compositions according to entries 4, 6, 8 and 9 are according to the invention. Compositions according to entries 1 to 3, 5, 7, 10 and 11 are for comparison.

The results are shown in Table 1.

**Table 1 Efficiency of various compositions**

| **Decontamination Efficiency, %** | **Removes Soot** | **Ingredient 1** | **wt%** | **Ingredient 2** | **wt%** | **Ingredient 3** | **wt%** | **Ingredient 4** | **wt%** |
|---|---|---|---|---|---|---|---|---|---|
| Entry 1: 100 (b) | - | BSA | 10 | Norit^{®} B test; activated carbon | 10 | Kaolin | 10 | Bentonite | 10 |
| Entry 2: 61.7 (b) | - | Poloxamer 407 | 10 | Norit^{®} B test; activated carbon | 10 | Kaolin | 10 | Bentonite | 10 |
| Entry 3: 66.6 (b) | + | Chitosan | 2 | Norit^{®} B test; activated carbon | 10 | Kaolin | 10 | Bentonite | 10 |
| Entry 4: 82.3 (f) | + | Soybean protein isolate | 5 | Bentonite | 10 | Kaolin | 10 | - | - |
| Entry 5: 80.4 (b) | - | Soybean protein isolate | 5 | Bentonite | 10 | Kaolin | 10 | Norit^{®} B test; activated carbon | 10 |
| Entry 6: 97.2 (b) | + | Soybean protein isolate | 5 | Bentonite | 10 | Kaolin | 10 | - | - |
| Entry 7: 81.3 (b) | - | Soybean protein isolate | 5 | Bentonite | 10 | Kaolin | 10 | Norit^{®} B test; activated carbon | 10 |
| Entry 8: 94.9 (f) | + | Bentonite | 15 | Kolliphor^{®}P407 | 8 | Kaolin | 12 | - | - |
| Entry 9: 86.8 (b) | + | Bentonite | 15 | Kolliphor^{®}P407 | 8 | Kaolin | 12 | - | - |
| Entry 10: 53,5 (b) | - | Roquette DS112 | 2 | Norit^{®} B test; activated carbon | 10 | Kaolin | 10 | Bentonite | 10 |
| Entry 11: 58,7 (B) | - | Chitosan | 2 | Norit^{®} B test; activated carbon | 10 | Kaolin | 10 | Bentonite | 10 |

| | | | | | | | | | |
|---|---|---|---|---|---|---|---|---|---|
| (b) = benzo[a]pyrene, (f) = fluorene; + denotes sufficient removal, - no/insufficient removal | | | | | | | | | |

The results in Table 1 show that the compositions according to the invention are highly effective in decontaminating PAH and soot showing decontamination rates of 82.3 % up to 97.2 %.

### Example 4: Decontamination efficiency of commercial products (Comparative)

Decontamination experiments using commercial products were performed as described in Example 2.

**Table 2: Efficiency of different decontamination compositions**

| **Decontamination Efficiency of PAH, %** | **Removes Soot** | **Product** |
|---|---|---|
| 100 (f) | - | WO 2021/214209 |
| 100 (b) | - | WO 2021/214209 |
| 76.0 (b) | + | commercial cleaning agent composed of water, C₁₆₋₂₀ isoalkane, Juglans regia shell powder, sodium laureth sulfate, sulfated castor oil, sodium chloride, cocamidopropyl betaine, quaternium-18 bentonite, titanium dioxide, PEG-4 rapeseed amide, potassium sorbate, cellulose gum, sodium benzoate, citric acid. |
| 60.0 (b) | + | handsoap |
| 0 (b) | - | commercial decontamination agent composed of water and polyvalent ingredients |
| 51,7 (b) | - | water |
| 5.0 (f) | - | water |

| | | |
|---|---|---|
| (b)= benzo[a]pyrene, (f)= fluorene; + denotes sufficient removal, - no/insufficient removal | | |

The measurements in Table 2 show that washing with water removes only 5.0% of the PAH from the skin, whereas treatment with water and soap removes 60% of the PAH. Contrary to this and as shown in Table 1 above, the compositions of the invention were able to remove soot and up to 97.2% of the PAH from the skin, and thus, were more efficient than soap and water. They were also more efficient than other known professional (commercial) products used for decontamination of the skin (see Table 2). 21

## Claims

1. Composition, comprising
(A) water;
(B) at least one phyllosilicate;
(C) at least one water-soluble or water-dispersible polymer; and
(D) optionally at least one thickening agent;
wherein the composition contains less than 1 % by weight charcoal and/or graphite and/or another carbon-based powder, preferably wherein the composition does not contain charcoal and/or graphite and/or another carbon-based powder.

2. Composition of claim 1, wherein
(C) is or comprises at least one water-soluble or water-dispersible polyether or protein or a combination thereof.

3. Composition of claim 1 or 2, wherein
(D) the at least one thickening agent is selected from the group consisting of polysaccharide, cellulose ether, and polymer based on polyacrylic acid.

4. Composition of any one of the preceding claims, wherein
(B) is or comprises a combination of at least two different phyllosilicates; preferably wherein
(B) is or comprises a sodium aluminium silicate or an aluminium silicate or a combination thereof.

5. Composition of any one of the preceding claims, wherein
(B) is or comprises bentonite or kaolinite or a combination thereof.

6. Composition of any one of the preceding claims, wherein
(C) is or comprises a poloxamer or a poloxamine or a soybean protein or a combination of two or more thereof.

7. Composition of claim 6, wherein the poloxamer has the CAS no. 691397-13-4 or 9003-11-6 or the soybean protein is soybean protein isolate.

8. Composition of any one of the preceding claims, wherein
(D) is or comprises a cellulose ether or a polysaccharide or a combination thereof.

9. Composition of any one of the preceding claims, wherein
(D) is or comprises a polysaccharide selected from xanthan, chitosan, dextran, guar gum, polygeline, pectin and carrageenan.

10. Composition of any one of the preceding claims, comprising
(A) from 20 to 90 % by weight;
(B) from 1 to 40 % by weight;
(C) from 1 to 40 % by weight;
(D) from 0 to 10 % by weight;
based on the total weight of (A) to (D) (100 % by weight);
preferably comprising
(A) from 30 to 80 % by weight;
(B) from 5 to 30 % by weight;
(C) from 5 to 30 % by weight;
(D) from 0 to 10 % by weight;
based on the total weight of (A) to (D) (100 % by weight).

11. Cleaning composition comprising or consisting of the composition as defined in any one of the preceding claims.

12. Cleaning composition of claim 11, comprising from 40 to 100 % by weight of the composition defined in any one of claims 1 to 10.

13. Use of a composition as defined in any one of the preceding claims for removing polycyclic aromatic hydrocarbons or soot or polycyclic aromatic hydrocarbons and soot from human skin.

14. Method of cleaning human skin, wherein said skin is contaminated with a polycyclic aromatic hydrocarbon or soot or polycyclic aromatic hydrocarbons and soot, comprising steps (A) and (B) and optionally step (C):
(A) contacting said skin with a composition as defined in any one of claims 1 to 12;
(B) rinsing the contacted skin of step (A) with water;
(C) optionally repeating steps (A) and (B).

15. Use of claim 13 or method of claim 14, wherein said polycyclic aromatic hydrocarbon is comprised in soot.
